# EUROPEAN PATENT SPECIFICATION

(11) **EP 1 206 530 B1**
(45) Date of publication and mention of the grant of the patent: **17.06.2009**
(21) Application number: 00955297.7
(22) Date of filing: 01.08.2000
(51) Int. Cl.: C12N 15/02, C12N 15/06, C12N 15/08, C07K 16/00, C12P 21/08, G01N 33/53, A61K 39/00, C07K 16/40, C12N 9/02

(54) **Measuring human cytochrome P4503A4 (CYP3A4)**
Bestimmung humanes Cytochrom P450 3A4 (CYP3A4)
Mésuration du cytochrome p450 3A4 humain (CYP3A4)

(30) Priority: 05.08.1999 US 147315 P; 13.09.1999 US 153638 P
(43) Date of publication of application: 22.05.2002
(60) Divisional application: 08105435.5
(73) Proprietor: Merck & Co., Inc., Rahway, New Jersey 07065-0907 (US)
(72) Inventor: MEI, Qin, Rahway, NJ 07065-0907 (US); RUSHMORE, Thomas, H., Rahway, NJ 07065-0907 (US); SHOU, Magang, Rahway, NJ 07065-0907 (US)
(74) Representative: Horgan, James Michael Frederic
(86) International application number: PCT/US2000/020864
(87) International publication number: WO 2001/011035

(56) References cited:
- WO-A-97/19112
- WO-A-98/44939
- US-A- 5 300 435
- US-A- 5 650 295
- US-A- 5 658 913
- SHIMOJIMA MASAYUKI ET AL: "Generation of monoclonal antibodies against a feline CD antigen (CD4) expressed by a recombinant baculovirus." JOURNAL OF VETERINARY MEDICAL SCIENCE, vol. 59, no. 6, 1997, pages 467-469, XP002235495 ISSN: 0916-7250
- MARIANI ET AL: "Detection of active infection of Sf9 insect cells by recombinant baculoviruses" JOURNAL OF VIROLOGICAL METHODS, AMSTERDAM, NL, vol. 69, 1997, pages 19-28, XP002095592 ISSN: 0166-0934
- DELENDA C ET AL: "ANALYSIS OF C-TERMINALLY TRUNCATED DENGUE 2 AND DENGUE 3 VIRUS ENVELOPE GLYCOPROTEINS: PROCESSING IN INSECT CELLS AND IMMUNOGENIC PROPERTIES IN MICE" JOURNAL OF GENERAL VIROLOGY, SOCIETY FOR GENERAL MICROBIOLOGY, READING, GB, vol. 75, no. 7, 1 July 1994 (1994-07-01), pages 1569-1578, XP002009563 ISSN: 0022-1317
- MEI QIN ET AL: "Role of a potent inhibitory monoclonal antibody to cytochrome P-450 3A4 in assessment of human drug metabolism." JOURNAL OF PHARMACOLOGY AND EXPERIMENTAL THERAPEUTICS, vol. 291, no. 2, November 1999 (1999-11), pages 749-759, XP002235497 ISSN: 0022-3565
- GELBOIN H V ET AL: "INHIBITORY AND NON-INHIBITORY MONOCLONAL ANTIBODIES TO HUMAN CYTOCHROME P450 3A3/4" BIOCHEMICAL PHARMACOLOGY, PERGAMON, OXFORD, GB, vol. 50, no. 11, 1995, pages 1841-1850, XP000644797 ISSN: 0006-2952
- YANG TIAN J ET AL: "Eight inhibitory monoclonal antibodies define the role of individual P-450S in human liver microsomal diazepam, 7-ethoxycoumarin and imipramine metabolism." DRUG METABOLISM AND DISPOSITION, vol. 27, no. 1, January 1999 (1999-01), pages 102-109, XP002235496 ISSN: 0090-9556
- GELBOIN H V: "CYTOCHROME P450 AND MONOCLONAL ANTIBODIES" PHARMACOLOGICAL REVIEWS, WILLIAMS AND WILKINS INC., BALTIMORE, MD,, US, vol. 45, no. 4, 1993, pages 413-453, XP002951949 ISSN: 0031-6997
- KRAUSZ ET AL: "Inhibitory monoclonal antibodies specific for human cytochrome P450 2C8 and 2C9 define their roles in human liver microsomal metabolism" FASEB JOURNAL, FED. OF AMERICAN SOC. FOR EXPERIMENTAL BIOLOGY, BETHESDA, MD, US, vol. 13, no. 5, PART 2, 15 March 1999 (1999-03-15), page A811 XP002140770 ISSN: 0892-6638
- GELBOIN H V ET AL: "Inhibitory monoclonal antibodies to human cytochrome P450 enzymes: a new avenue for drug discovery" TRENDS IN PHARMACOLOGICAL SCIENCES, ELSEVIER TRENDS JOURNAL, CAMBRIDGE, GB, vol. 20, no. 11, 1 November 1999 (1999-11-01), pages 432-438, XP004182934 ISSN: 0165-6147
- YAMAZAKI ET AL.: 'Different contributions of cytochrome P450 2C19 and 3A4 in the oxidation of omeprazole by human liver microsomes: Effects of contents of these two forms in individual human samples' THE JOURNAL OF PHARMACOLOGY AND EXPERIMENTAL THERAPEUTICS vol. 283, no. 2, 1997, pages 434 - 442, XP002924291
- KOBAYASHI ET AL.: 'Identification of cytochrome P450 isoforms involved in citalopram N-demethylation by human liver microsomes' THE JOURNAL OF PHARMACOLOGY AND EXPERIMENTAL THERAPEUTICS vol. 280, no. 2, 1997, pages 927 - 933, XP002924271
- PAN ET AL.: 'Characterization of the cytochrome P450 isoenzymes involved in the in vitro N-dealkylation of haloperidol' BR. J. CLIN. PHARMACOL. vol. 44, 1997, pages 557 - 564, XP002924251
- DANIELSON ET AL.: 'Molecular cloning of a family of xenobiotic-inducible drosophilid cytochrome P450s: Evidence for involvement in host-plant allelochemical resistance' PROC. NATL. ACAD. SCI. USA vol. 94, September 1997, pages 10797 - 10802, XP002924292
- TAN ET AL.: 'Expression and purification of a secreted functional mouse/human chimaeric antibody against bacterial endotoxin in baculovirus-infected insect cells' BIOTECHNOL. APPL. BIOCHEM. vol. 30, 29 July 1999, pages 59 - 64, XP002924272
- PARK S.S. ET AL: 'Monoclonal antibodies to phenobarbital-induced rat liver cytochrome P-450' BIOCHEM. PHARMACOL. vol. 33, no. 13, 1984, pages 2071 - 2081
- CHOU M. ET AL: 'Antibodies against the calcium-binding protein calsequestrin from Streptanthus tortuosus (Brassicacee).' PLANT PHYSIOL. vol. 91, 1989, pages 1259 - 1261
- MEIKLE P.J. ET AL: 'The beta-glucan synthase from Lolium multiflorum' J. BIOL. CHEM. vol. 266, no. 33, 1991, pages 22569 - 22581
- CHICHEPORTICHE Y. ET AL: 'Characterization of cytoplasmically oriented Golgi Proteins with a monoclonal antibody.' J. CELL BIOL. vol. 99, December 1984, pages 2200 - 2210

## Description

### FIELD OF THE INVENTION

The present invention relates to measuring CYP3A4 using a monoclonal antibody (MAb) specific for human cytochrome P450 3A4 (CYP3A4).

### BACKGROUND OF THE INVENTION

Although the precise number of cytochrome P450 isoforms is unknown, there are at least fifteen forms of basal and inducible P450s derived from P450 families 1, 2, and 3, which play a role in the metabolism of most xenobiotics. The P450s identified so far are represented by multiple forms, and some exist at very low concentrations in tissues. Their substrate and product specificity often overlap. In addition, the levels of many P450s change in response to environmental, hormonal or nutritional influences, or are determined by genetic polymorphism. These factors have greatly limited our understanding of the precise role of individual cytochrome P450s in the metabolism, activation and detoxification of different cytochrome P450 substrates and, further, have prevented our understanding of the relationship between P450 phenotype in various tissues and an individual's responsiveness and sensitivity to drugs.

Developing reagent(s) such as inhibitory monoclonal antibodies that could precisely define a role of a single P450 molecule or even a class of P450 molecules in the metabolism of a particular drug would be of tremendous benefit to the field of drug metabolism. Monoclonal antibodies (MAbs) are ideally suited for these types of inquiries (i.e., the detailed analysis of specific molecules, such as the cytochrome P450 molecules above) in that they are chemically pure reagents that recognize a single antigenic determinant or epitope on the surface of a P450 molecule resulting in the inhibition of the catalytic activity associated with same. The measurement of P450 inhibition in different tissue preparations, thus, allows one to estimate the contribution of a particular P450 to the metabolism of various compounds in the tissue preparations, e.g., in human liver microsomes containing multiple P450s.

Towards this end, a hybridoma clone producing MAbs (MAb-A334™, IgM) that inhibit the enzymatic activity of cytochrome P450 3A4 (CYP3A4) probed with various substrates was developed (Gelboin et al., 1995 Biochem. Pharmacol. 50(11):1841-1850). The MAbs, however, displayed only partial inhibition (45∼90%) of most recombinant CYP3A4-mediated reactions (up to 100 µl ascites/mL incubation), and the inhibitory cross-reactivity of the MAb with other subfamilies of P450 was not fully investigated by using individual recombinant P450s, or P450-marker assays with human liver microsomes. Further, the MAb exhibited no immunoblot activity for CYP3A4 antigen on a Western blot impairing the use of same to characterize the quantitative role of CYP3A4 in the metabolism of a given drug. Instead, Gelboin proposes a supplementing monoclonal antibody, MAb 275-1-2, which, while lacking any inhibitory activity against CYP3A4, provides a Western blot against CYP3A4.

CYP3A4 remains of considerable interest and represents one of the best-studied members of the human P450 superfamily. Interest has focused on this P450 due to its abundance in human liver, inducibility by numerous agents (e.g., glucocorticoids, phenobarbital, etc.), and because of its prominent role in the metabolism of a large number of clinically relevant drugs.

A monoclonal antibody specific for CYP3A4, which both provides an immunoblot reaction against CYP3A4 and effectively inhibits CYP3A4-mediated reactions would contribute greatly towards the phenotyping of CYP3A4 in preclinical and clinical drug metabolism. Such a MAb could be ideally used in assays to assess the contribution of a particular P450 to the metabolism of a suspect drug in human tissue preparation. Further, said MAb could be used to determine the expression level of CYP3A4 in various tissues by a variety of immunoassays, including ELISA, western blot and immunohistochemical methods.

### SUMMARY OF THE INVENTION

The present invention relates to using a monoclonal antibody specific for human cytochrome P450 3A4 which was developed by the fusion of myeloma tumor cells with isolated B-lymphocytes from spleens of mice immunized with baculovirus-expressed human CYP3A4.

The monoclonal antibody is specific for human CYP3A4 of the isotype IgG₂ₐ wherein the antibody exhibits cross-reactivity with human CYP3A5, human fetal CYP3A7 and Rhesus monkey testosterone 6β-hydroxylase but does not cross-react with human CYP1A1, CYP1A2, CYP2A6, CYP2B6, CYP2C8, CYP2C9, CYP2C19, CYP2D6, CYP2E1 or testosterone 6β-hydroxylase in dog, rat and mouse liver microsomes. The antibody in accordance with this description is MAb 10-1-1 as illustrated in the examples. This antibody was deposited with the American Type Culture Collection (ATCC), 10801 University Boulevard, Manassas, VA 20110-2209, in accordance with the Budapest Treaty on July 14, 1999 and assigned an accession number of PTA-351.

Notably, only 1 and 5 µl (0.5 µM and 2.5 µM of IgG₂ₐ) of the MAb mouse ascites in one mL incubation containing 20 pmol of CYP3A4 inhibited strongly the testosterone 6β-hydroxylation by 95% and 99%, respectively, rendering the MAb described herein a potent inhibitor of CYP3A4-catalyzed reactions. Inhibition of human CYP3A4 activity is measured by the inhibition of CYP3A4-catalyzed reactions such as testosterone 6β-hydroxylation, diazepam 3-hydroxylation and N-demethylation. Reactions catalyzed by CYP3A4 are known in the art as are methods by which to measure the resultant degree of metabolism. One way by which the degree of metabolism could be measured is by measuring the substrate and product levels after stopping the reaction and comparing said levels with a control sample wherein antibody was not added. For example, the reaction could be stopped by the addition of dichloromethane, the metabolites then extracted from the aqueous phase to the organic phase, the organic phase dried out, and the residue of the sample applied to a High Performance Liquid Chromatograph (HPLC) immediately for separation and quantitation of metabolites. Other methods for determining the level of metabolism are also acceptable and included herein.

The MAb is produced from a hybridoma developed by the fusion of myeloma tumor cells with isolated B-lymphocytes from spleens of mice immunized with insect cell microsomes comprising baculovirus-expressed antigen. Immunization involves injecting the antigen (in this case, the microsomal preparation) into an antibody-producing species, preferably a mammal and, more preferably, a mouse. Usually an initial injection is followed by subsequent booster injections to maximize the response. The amount of antigen injected must be adequate to elicit a sufficient amount of antibody to be detectable. Balb/c mice are immunized intraperitoneally with 50 µg of Sf21 cell microsomes containing baculovirus-expressed antigen (of approximately 29% purity) emulsified in 0.2 mL of complete Freund's adjuvant (first immunization), followed by two booster injections with incomplete Freund's adjuvant on the 10^{th} and 20 ^{th} days. Following immunization, the animal will produce B-cells that produce and secrete antibodies specific for the antigen. These cells can be isolated by removal of the spleen of said animal. Fusion of the antibody-producing cells to a suitable stable and long living cell line (i.e., immortal cell line such as myeloma tumor cells) is done according to techniques that are known in the art. Most preferably, fusion is performed with PEG 5000 as described in (Park et al., 1986 Biochem. Pharmacol. 35:2859-2867).

The insect used is *Spodoptera frugiperda.*

Screening techniques to select a hybridoma colony consisting of cells that produce the desired antibody are known in the art. Positive clones are selected by comparison with microsomes of cells infected with wild type baculoviruses, the positive clones evidenced by a notable increase in absorption at 405 nm. Selected positive hybridomas are then transferred to 24-well plates for further growth until confluence and the media tested for ELISA and inhibitory activity by HPLC or LC-MS for assurance of the desired MAbs excreted from cloned hybridomas. Hybridoma cells producing such inhibitory MAbs are then subcloned at least three times and the media tested at each time.

Hybridomas producing specific monoclonal antibodies can be made by immunizing mice with microsomal proteins comprising baculovirus-expressed antigen, and fusing isolated B-lymphocytes from spleens of the immunized mice with myeloma tumor cells.

Ascites fluid comprising monoclonal antibodies specific for a particular antigen can be made by immunizing mice with microsomal proteins comprising baculovirus-expressed antigen, fusing isolated B-lymphocytes from spleens of the immunized mice with myeloma tumor cells, identifying those hybridomas producing antibodies which are reactive to the antigen and which inhibit reactions catalyzed by the antigen, injecting the hybridomas into mice, and extracting the ascites fluid. Means by which one of ordinary skill in the art can identify antibodies reactive to the antigen are available in the art and well known, e.g., ELISA, Western blot, etc. Methods of determining whether an antibody is capable of inhibiting a particular reaction catalyzed by its respective antigen are also known in the art and involve carrying out the reaction both in the presence and in the absence of the antibody and determining the levels of substrate and product produced, the level of product produced indicating the level of metabolism had (i.e., a lower level of substrate-to-product conversion in the sample comprising the antibody indicating the presence of inhibition).

An embodiment of the invention is a method of determining expression levels of cytochrome P450 3A4 in a sample which comprises contacting the sample purportedly containing cytrochrome P450 3A4 with the above antibody specific to the cytochrome having a detectable label, and measuring the amount of label in said sample; said amount indicating the level of expression of CYP3A4 in the sample. Detectable labels are labels which can be detected. Labels suitable in this process are known in the art and include fluorescent labels, radioactive labels, and enzymes wherein a substrate is metabolized to a quantifiable product (e.g., wherein the enzyme is alkaline phosphatase and the substrate is 5-bromo-4-chloro-3-indolyl-phosphate/nitroblue tetrazolium).

A preferred method of determining expression levels of cytochrome P450 3A4 in a sample comprises separating a sample on a gel such as an SDS polyacrylamide gel, transferring same to a piece of material capable of adsorbing the separated sample (e.g., nitrocellulose paper), contacting the separated sample on the material with a first antibody in accordance with the above description specific for said cytochrome, contacting the material with a second antibody specific for the first antibody which comprises a detectable label (see above) allowing for the quantitation of the bound first antibody (e.g., an alkaline phosphatase-conjugated anti-mouse IgG (H+L) for CYP3A4); and quantitating the level of antibody, same indicating the level of antigen. This last step would include, for example, treating the alkaline phosphatase-conjugated anti-mouse IgG (H+L) on the adsorbed material with a substrate for the alkaline phosphatase, e.g., 5-bromo-4 chloro-3-indolyl-phosphate/nitroblue tetrazolium, the metabolism of same being identified by a colored product which is to be quantitated to determine the level of antigen.

Another embodiment is a method of identifying CYP3A4-catalyzed metabolism of a substrate, which comprises contacting a sample comprising both CYP3A4 and the above antibody with the substrate, measuring both substrate and product levels, and comparing the substrate and product levels with levels obtained from a control sample comprising both CYP3A4 and the substrate but not the antibody; a lower level of substrate-to-product conversion in the sample comprising the antibody indicating the presence of CYP3A4-catalyzed metabolism. Thus, inhibition of CYP3A4-catalyzed metabolism by the antibody is present when the level of substrate-to-product conversion is less in the sample comprising the antibody; "substrate-to-product conversion" being the metabolism of the substrate to the product.

Yet another embodiment is a method of quantifying CYP3A4-specific metabolism of a substrate in a sample which comprises contacting a sample comprising both the above antibody specific to the cytochrome employed and cytochrome P450s known to be involved in the metabolism of the substrate with the substrate, measuring substrate and product levels, and comparing the substrate and product levels with levels obtained from a control sample comprising CYP3A4 and the substrate but not the antibody. The degree of variation, in this case, indicates the contribution of CYP3A4 to the metabolism of that particular substrate in the tissue preparation studied, be it human or other. "CYP3A4-specific metabolism" in this respect, thus, means only that metabolism wherein CYP3A4 is the responsible agent, and not other CYP3A4s which may be present in the sample. To enhance this method, antibodies to other CYP3A4s could also be used either in conjunction with the above antibodies or separate. In this manner, the particular contributions of each cytochrome P450 could be quantified and analyzed. This is extremely useful in situations where a compound is metabolized by more than one P450.

### BRIEF DESCRIPTION OF THE DRAWINGS

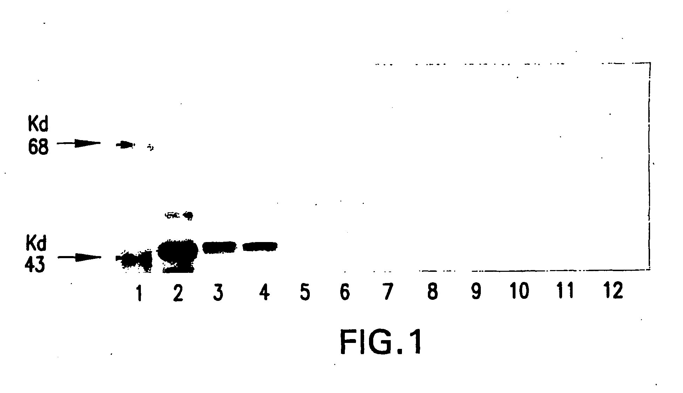

Figure 1 shows cross-reactivity of MAb_{3A4a} towards eleven recombinant P450s by western immunoblotting. Each lane contained 1 pmol of P450. Lane 1: molecular markers; lane 2: CYP3A4; lane 3: CYP3A5; lane 4: CYP3A7; lane 5: CYP1A2; lane 6: CYP2A6; lane 7: CYP2B6; lane 8: CYP2C8; lane 9: CYP2C9; lane 10: CYP2C19; lane 11: CYP2D6 and lane 12: CYP2E1.

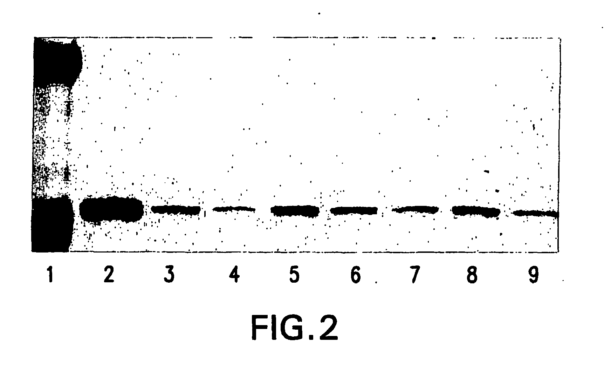

Figure 2 shows westernblot analyses of CYP3A4/5 in liver microsomes from seven individuals. 1 pmol of total P450 from each sample was loaded on the gel. Lane 1: molecular markers; lane 2: baculovirus-expressed CYP3A4; lane 3: L14; lane 4: L15; lane 5: L16; lane 6: L17; lane 7: L21; lane 8: L22 and lane 9: PH (pooled liver microsomes).
Figure 3 shows westernblot analyses of P450 present in liver microsomes from human, Rhesus monkeys, dogs, rats and mice. 1 Pmol of CYP3A4 and 5 pmol of microsomal P450s in human and animal livers were loaded on the gel. Lanes 1 and 9: molecular markers; lanes 2 and 8: CYP3A4; lane 3: human; lane 4: monkey; lane 5: dog; lane 6: rat and lane 7: mouse.
Figure 4 shows mAb_{3A4a} inhibition of testosterone metabolism by baculovirus-expressed CYP3A4, CYP3A5 and CYP3A7.
Figure 5 shows mAb_{3A4a} inhibition of diazepam metabolism catalyzed by recombinant CYP3A4.
Figure 6 shows inhibition comparison of MAb_{3A4a} with MAb-A334™ in the metabolism of testosterone catalyzed by recombinant CYP3A4.
Figure 7 shows the specificity of MAb_{3A4a} inhibition with twelve recombinant P450s.
Figure 8 shows the cross-reactivity of MAb_{3A4a} inhibition with P450-specific reactions catalyzed by human liver microsomes.
Figure 9 illustrates the metabolic pathways of diazepam by cytochrome P450s.

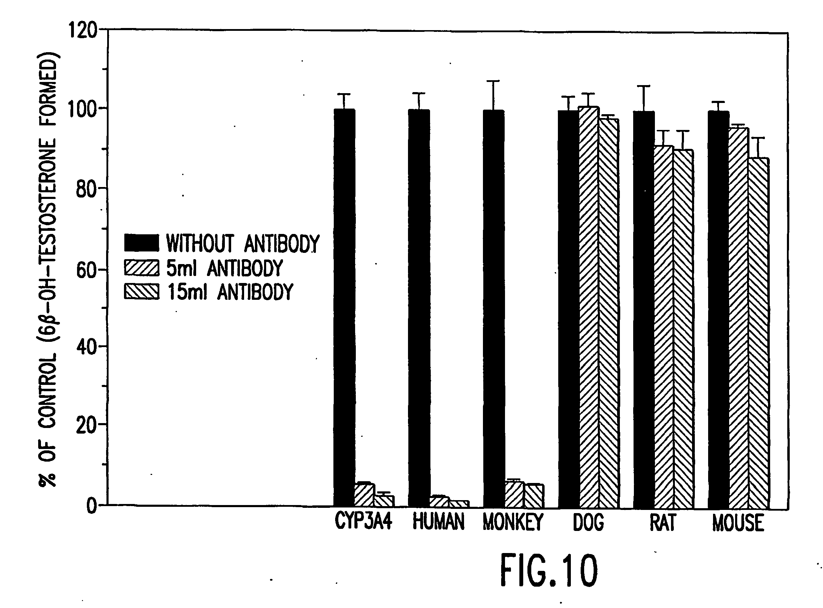

Figure 10 shows the effect of MAb_{3A4a} (0, 5 and 15 □1, blackened, grayed and white blocks, respectively) on testosterone-6□-hydroxylation catalyzed by recombinant CYP3A4, human, monkey, dog, rat and mouse liver microsomes.
Figure 11 shows an assessment of CYP3A and CYP2C contribution to diazepam 3-hydroxylation in liver microsomes of five samples.
Figure 12 shows an assessment of CYP3A and CYP2C contribution to diazepam *N*-demethylation in liver microsomes of five samples.

### DETAILED DESCRIPTION OF THE INVENTION

As used throughout the specification and claims, the following definitions shall apply:

The terms "specific" or "specificity" as used herein regarding antibodies against human CYP3A4 mean that these antibodies do not cross-react with the major human cytochrome P450 isoforms in the 1A, 2A, 2C, 2D, 2E, 3A subfamilies.

The terms "substantially inhibit", "inhibit", etc. as used herein regarding antibodies against human CYP3A4 mean that these antibodies inhibit at least about 80-95% of human CYP3A4 activity *in vitro.*

The terms "MAb_{3A4a}" and "MAb10-1-1" and "PTA-351" are synonymous for Applicants' monoclonal antibody as described herein.

A monoclonal antibody specific for cytochrome P450 3A4 (MAb_{3A4a}) of the isotype IgG₂ₐ has been identified. MAb_{3A4a} is directed at a single epitope on the antigen, cytochrome P450 3A4, allowing for the high specificity noted of monoclonal antibodies.

In order to obtain the above antibody, microsomes of *Spodoptera frugiperda* (Sf21) insect cells infected with recombinant baculoviruses encoding human CYP3A4 cDNA were used to immunize mice. Spleen cells from the immunized mice were then fused to myeloma cells, and hybridoma cells producing the monoclonal antibodies of the instant invention were identified using ELISA; identification being based on binding to microsomes containing CYP3A4 antigen. This involved subtracting out those non-specific antibodies with absorption at 405nm. Positive hybridoma clones were then tested against testosterone metabolism (6β-hydroxylation) to identify those clones effective in inhibiting CYP3A4-catalyzed testosterone metabolism. Following subcloning to obtain monoclonality, the resulting hybridoma cells of interest were injected into mice for the preparation of ascites fluid. Resultant ascites fluid exhibited a strong binding activity on immunoblot with CYP3A4, with a lesser response to CYP3A5 and CYP3A7. MAb_{3A4a} did not cross-react with any of the other eight human P450s tested (CYP1A2, CYP2A6, CYP2B6, CYP2C8, CYP2C9, CYP2C19, CYP2D6, and CYP2E1).

CYP3A4 within insect microsome preparations served as both immunogen and cloning component of the hybridoma technology. The implications arising from this finding are significant. Applicants have found that, through the use of relatively impure antigen preparations in both immunization and cloning, one could obtain MAbs to P450 proteins which may be present at low concentrations or minor deviants of the primary protein in the immunogen preparation. Further, this method is not limited to antibodies of P450 molecules. Antibodies to other molecules of interest could be identified via this process as well. Accordingly, the previously reported procedures involving purification of large amount of P450 proteins in order to isolate MAbs are no longer necessary (Gelboin *et al*., 1995; Gelboin et al., 1997 Pharmacogenetics 7:469-477).

The resulting antibody in the instant case, MAb_{3A4a}, was shown to have both a strong ELISA and western blot with CYP 3A4 antigen. The positive westernblot result indicates that the epitope on CYP3A4 recognized by MAb_{3A4a} is of the native conformation of the protein. MAb_{3A4a} can thus be used beneficially as a sensitive probe for the quantitation of the specific CYP3A4 in human tissue with a sensitivity of 0.1 pmol of CYP3A4 being able to be reached on westernblot using alkaline phosphatase-conjugated anti-mouse IgG (H+L).

The antibody used in the instant invention possesses many advantages over that disclosed in the art. For instance, roughly 80 µL of MAb-A334™ mouse ascites (Gelboin) per mL incubation is required to obtain inhibition of CYP3A4-mediated testosterone-6β-hydroxylation by 79%. By contrast, only 0.5 µL of MAb_{3A4a} ascites, a preparation of Applicants' monoclonal antibody, is required to achieve that percent inhibition (82%).

The antibody of the instant invention cross-reacts to a lesser extent with CYP3A5 and CYP3A7. This most likely reflects truly shared antigenic determinants on the surface of the antigens, especially given the highly conserved homology amongst CYP3A5/7 and CYP3A4, 84% and 88%, respectively. A monoclonal antibody like that of the instant invention which cross-reacts with CYP3A7 is a benefit for identification of CYP3A7 in human fetal liver in which CYP3A7 is a sole CYP3A subfamily member. Further, cross-reactivity of MAb_{3A4a} with CYP3A5 can be utilized to assess quantitatively the total contribution of CYP3A subclass to *in vitro* drug metabolism, although the expression level of CYP3A5 in liver is insignificant.

Notably, MAb_{3A4a} immunoblots microsomal P450(s) and strongly inhibits testosterone 6β-hydroxylase activity present in humans and monkey, but not in dog, rat, or mouse. The cross-reactivity of MAb_{3A4a} in closely related species is probably attributed to the highly conserved CYP3A sequences between human and monkey, in which CYP3A4 exhibits 93% identity of amino acid sequence with CYP3A8 (monkey), but to a lesser extent, 80% with CYP3A12 (dog), 73% and 72% with CYP3A1/2 in rat, and 73% with CYP*3a11* in mouse. Thus, MAb_{3A4a} directed against one CYP3A4 generally cross-reacts not only with other forms (CYP3A5 and CYP3A7) in the same species but also with other CYP3A forms in monkey. Consequently, cross-reactivity of the MAb with other species can provide an additional usefulness in the identification of CYP3A(s).

MAb_{3A4a} is, further, uniquely suited for reaction phenotyping, i.e., determining the contribution of epitope-specific CYP3A to metabolic reactions and for immuno-quantitation of CYP3A expression in human tissues. In addition, the cross-reactivity of MAb_{3A4a} with other CYP3As in both inhibitory and westernblotting activity can be very useful for the study of CYP3A7 in human fetal and CYP3A in monkey.

The following non-limiting examples are presented to better illustrate the invention.

### EXAMPLE 1

### Chemicals

The following chemicals were obtained from commercial sources: (±)-4-hydroxymephenytoin, (±)-mephenytoin, 6-hydroxychloraoxazone, (±)-bufuralol hydrochloride salt, and (±)-1'-hydroxybufuralol maleate salt from ULTRAFINE Chemicals (Manchester, UK); diazepam (DZ), temazepam (TMZ), nordiazepam (NDZ), flurbiprofen, chlorzoxazone, taxol, coumarin, 7-hydroxycumarin, phenacetin, acetaminophen, and NADPH from Sigma (St. Louis, MO); testosterone, 6β-hydroxytestosterone, estrone, 2-hydroxyestrone, 6β-hydroxyprogesterone from Steraloids Inc. (Wilton, NH); MAb-A334™ (Cat. No =A334, anti-CYP3A4MAb), human CYP3A5+OR supersomes, 6-hydroxychlorzoxazone and 6α-hydroxypaclitaxol from Gentest Corp. (Woburn, MA); benzo[*a*]pyrene *trans*-4,5-dihydrodiol (B[*a*]P *t*-4,5-diol), phenanthrene, 9-hydroxyphenanthrene and benz[*a*]anthracene *trans*-5,6-dihydrodiol (BA t-5,6-diol) from the National Cancer Institute Chemical Carcinogen Repository (Kansas, MO); Sf-900II SFM, fetal bovine serum and HAT supplement from GIBCO (Frederick, MD); and 8-asaguanine resistant myeloma cell line, NS-1 from Frederick Cancer Research and Development Center (Frederick, MD).

### EXAMPLE 2

### Reparation of human P450s

Plasmids containing the full length cDNAs for P450s 3A4, 1A1, 1A2, 2A6, 2B6, 2C8, 2C9, 2C19, 2D6, 2E1, 3A5 and 3A7 and P450 oxidoreductase (OR) were provided from Dr. Frank J. Gonzalez of National Cancer Institute. These cDNAs are known in the art and are published as follows: Human CYP1A1 cDNA - Jaiswal et al., 1985 Science 228(4695):80-3, and Jaiswal et al., 1985 Nucleic Acids Res 13(12):4503-20; Human CYP1A2 cDNA - Jaiswal et al, 1986 Nucleic Acids Res 14(16):6773-4, and Jaiswal et al., 1987 J Exp Pathol 3(1):1-17; Human CYP2A6 cDNA- Yamano et al., 1990 Biochemistry 29(5):1322-9, and Yamano et al., 1989 Nucleic Acids Res 17(12):4888; Human CYP2B6 cDNA- Yamano et al., 1989 Biochemistry 28(18):7340-8; Human CYP2C8 cDNA - Kimura et al., 1987 Nucleic Acids Res 15(23):10053-4; Human CYP2C9 cDNA - Kimura et al., 1987 Nucleic Acids Res 15(23):10053-4; Human 2C19 cDNA - Romkes et al., 1991 Biochemistry 30, 3247-3277; Human CYP2D6 cDNA - Gonzalez et al., 1988 Nature 331(6155):442-6; Human CYP2E1 cDNA - Song et al., 1986 J. Biol. Chem. 261(35):16689-97 [published erratum appears in J Biol Chem 1987 Jun 25;262(18):8940]; Human CYP3A4 cDNA - Gonzalez et al., 1988 DNA 7(2):79-86; Human CYP3A5 cDNA - Aoyama et al., 1989 J. Biol. Chem. 264, 10388-10395; and Human CYP3A7 cDNA - Kitada et al., 1987 J. Biol. Chem. 262, 13534-13537.

The entire coding region of each cDNA was excised from the vectors by digestion with respective enzymes and inserted into baculovirus shuttle vector, pBlueBac 4.5 (Invitrogen Corp., Carlsbad, CA), downstream of the polyhedrin promoter. Recombinant virus was constructed according to the manufacturer's procedure (Gonzalez et al., 1991 Methods Enzymol. 206:93-99) and isolated using Blue-Gal for color selection of recombinant virus. After two runs of plaque purification, the recombinant baculoviruses were propagated in *Spodoptera frugiperda* (*Sf21*) cells to generate high titer virus stocks for protein expression. *Sf21* insect cells (Invitrogen) were grown at 27°C in complete Sf900 SFM medium (BRL, Gaithersburg, MD) to a density of 1-2 x 106 cells/mL (400 mL in total) in 1-liter spinner flasks (Bellco Glass, Inc., Vineland, NJ) with enlarged blades at 90 rpm. Cells were infected at approximately 1.0 MOI (multiplicity of infection) of virus encoding individual P450s and 0.1 MOI of virus encoding OR. 1 µg hemin/mL medium in the form of a hemin-albumin complex was added. After 72 hours, cells were harvested by centrifugation and resuspended in 20% glycerol in 0.1 M KPi (potassium phosphate buffer) and stored at -70°C until microsomal preparation. The total P450 content was measured by the CO-difference spectrum. Microsomes were prepared as described below and the resulting protein concentration was determined with bicinchoninic acid according to the manufacturer's directions (Pierce Chemical Co., Rockford, IL). The activities of individual P450s co-expressed with OR were determined by the assays as indicated in Table 1 below. The assays of Table 1 are known in the art and published as follows: P450s 3A4, 3A5, and 3A7 by Hanioka et al., 1990 Protein Eng. 3:571-575; P450 1A1 by Shou et al., 1994 Cancer Lett. 83:305-313; P450 1A2 by Shou et al., 1997 Carcinogenesis 18:207-214; P450 2A6 by Yun et al., 1991 Mol. Pharmacol.40:679-685; P450 2B6 by Yang et al., 1998 Biochem. Pharmacol. 55:889-896; P450 2C8 by Rahman et al., 1994 Cancer Res. 54:5543-5556; P450 2C9 by Tracy et al., 1995 Biochem. Pharmacol. 49:1269-1275; P450 2C19 by Goldstein et al., 1994 Biochemistry 33:1743-1752; P450 2D6 by Crespi et al., 1995 Pharmacogenetics 5:234-243; and P450 2E1 by Peter et al., 1990 Chem. Res. Toxicol. 3:566-573.

**Table 1. Assays for individual P450s**

| P450 ^{a} | Substrate | Conc. (µM) | MAb (µl/mL) ^{b} | P450 Conc. (nM) | Product | Internal Standard^{c} |
|---|---|---|---|---|---|---|
| 3A4 | Testosterone | 200 | 10 | 15 | 6β-OH-testosterone | 6β-OH-progesterone |
| 3A5 | Testosterone | 200 | 10 | 15 | 6β-OH-testosterone | 6β-OH-progesterone |
| 3A7 | testosterone | 200 | 10 | 15 | 6β-OH-testosterone | 6β-OH-progesterone |
| 1A1 | phenanthrene | 300 | 10 | 25 | 9,10-dihydrodiol | B[*a*]P 9,10-diol |
| 1A2 | estrone | 100 | 10 | 50 | 2-OH-estrone | B[*a*]P cis-4,5-diol |
| 2A6 | coumarin | 100 | 10 | 20 | 7-OH-coumarin | 7-ethoxycoumarin |
| 2B6 | diazepam | 200 | 10 | 50 | Nordiazepam | 2-oxo-quanzepam |
| 2C8 | taxol | 25 | 10 | 25 | 6α-OH-taxol | Baccatin III |
| 2C9 | flurbiprofen | 20 | 10 | 15 | 4'-OH-flurbiprofen | B[*a*]A 5,6-diol |
| 2C19 | mephenytoin | 100 | 10 | 25 | 4'-OH-mephenytoin | Nirvanol |
| 2D6 | bufuralol | 25 | 10 | 10 | 1'-OH-bufuralol | Temazepam |
| 2E1 | chlorzoxazone | 200 | 10 | 50 | 6-OH-chlorzoxazone | B[*a*]A 5,6-diol |

| | | | | | | |
|---|---|---|---|---|---|---|
| ^{a.} Baculovirus-expressed individual P450s were co-expressed with OR in Sf21 insect cells as described in the text. ^{b.} MAb concentration was expressed as µL of mouse ascites per mL incubation mixture. ^{c.} Internal standard was used for metabolite quantitation. | | | | | | |

### EXAMPLE 3

### Microsomal preparation from human, animal livers and Sf21 cells expressing individual P450s

Microsomes of *Sf21* cells, human, Rhesus monkey, dog, rat and mouse livers were prepared by two different centrifugations (9,000g and 105,000g) as described previously (Wang et al., 1983 Biochemistry 22:5375-5383) and were reconstituted in a buffer (pH=7.4) containing 0.25 M sucrose, 1 mM EDTA, 0.5 mM DTT, 1.15% KCl and 0.1M KPi and stored at -80°C until used. Sf21 cell microsomes containing individual P450s with and without OR were used as a source of enzyme for metabolism study and immunogens for MAb production, respectively.

### EXAMPLE 4

### Procedure for MAb development

Three female Balb/c mice were immunized intraperitoneally with 50 µg of Sf21 cell microsomes containing baculovirus-expressed human CYP3A4 (29% purity) protein emulsified in 0.2 mL of complete Freund's adjuvant (first immunization), followed by two booster injections with incomplete Freund's adjuvant on 10^{th} and 20th days. Three days after the third injection, splenocytes of the mouse were taken for fusion with non-secreting myeloma cells P3/NSI/1-Ag4-1 (NS-1). Fusion of the spleen cells with the NS-1 cells was performed with PEG 5000 as previously described (Park et al., 1986 Biochem. Pharmacol. 35:2859-2867) and the fused cells were plated in microtiter wells in 96-well plate at a density of 1 x 104 cells per well and HAT medium was supplemented with 20% of FBS PRMI 1640 medium. Microtiter wells were examined daily for hybridoma growth. Three weeks later, when hybridoma cells approached confluence, media were tested with ELISA using baculovirus-expressed CYP3A4 as antigen (0.1 pmol/well). Positive clones (a total of 128 out of 1000 wells) were selected by comparison with microsomes of cells infected with wild type baculoviruses, which showed an approximate 3-fold increase in absorption at 405 nm with CYP3A4 (vs. wild type protein). Selected hybridomas were transferred to 24-well plates for further growth until confluence and then media were further tested for ELISA and inhibitory activity by HPLC or LC-MS for assurance of the desired MAbs excreted from cloned hybridomas. Of the 128 clones, one hybridoma clone (Mab_{3A4a}) resulted in potent inhibition of CYP3A4-catalyzed 6β-hydroxylation of testosterone(>93%); procedure below. Hybridoma cells producing such inhibitory MAbs were subcloned at least three times and their media were tested at each time.

### EXAMPLE 5

### ELISA (Enzyme-Linked Immunosorbent Assay)

ELISA was used for testing the binding activity of hybridoma supernatants collected from hybridoma screening (Gelboin *et al*., 1995). This procedure utilized alkaline phosphatase-conjugated goat F(ab')2 fragment to mouse IgG (H+L) from Cappel Research Products, and to mouse IgG (γ chain specific) or to mouse IgM (µ chain specific) from Jackson Immuno-Research Laboratories. Immunoassay plates were coated with Sf 21 microsomes containing baculovirus-expressed BYP3A4 at 1 pmol/well (100µL) or wild type baculovirus infected Sf21 cell microsomes as control in 1 X coating solution. As the hybrids began to grow, the spent medium from each well was screened for the presence of antibody

### EXAMPLE 6

### Immunoblot Assay

To determine the specificity and binding activity of MAbs, eleven baculovirus-expressed P450s and liver microsomes from human donors, Rhesus monkeys, dogs, rats and mice were subjected to SDS-PAGE on an 8% polyacrylamide gel for 2 hr at 120 volt (1 pmol/lane). Wild type Sf21 cell microsomes were also used as a negative control. The proteins were transferred to nitrocellulose filters using prechilled buffer containing 25 mM Tris, 200 mM glycine, and 20% methanol (pH=8). The nitrocellulose membranes were incubated for 2 hr in BSA blocking buffer (3% w/v BSA in TBS containing 0.05% v/v Tween-20) to reduce nonspecific binding and then washed twice for 5 min with TBS containing 0.05% v/v Tween-20. The ascites fluid containing MAbs was diluted in antibody buffer at a final titer of 1:1000 or 1:2000 and incubated with membrane for 3 hr at room temperature. The membranes were washed and protein bands were detected using alkaline phosphatase-conjugated anti-mouse IgG (H+L).

### EXAMPLE 7

### Production of mouse ascites containing Mab_{3A4a}

Large scale production of MAbs was performed by growing cloned hybridomas in culture. The MAb-forming hybridomas (1 x 10⁶ cells/mouse) were injected intraperitoneally into pristine-primed female BALB/c mice for production of concentrated MAbs. The ascites fluid was built up and withdrawn, two to three weeks after inoculation, and stored at -70°C (Park et al., 1986 Biochem. Pharmacol. 35:2859-2867). The protein concentration of this immunoglobulin preparation was 74 mg/mL mouse ascites.

### EXAMPLE 8

### Isotyping of mouse immunoglobulin (Ig)

Isotyping of MAbs was conducted by the Ouchterlony immunodiffusion using the mouse monoclonal antibody typing kits containing anti-mouse IgG₁, IgG₂ₐ, IgG_{2b}, IgG₃ and IgM from Binding Site, Inc. (Birmingham, AL). The murine immunoglobulin isotype of the Mab_{3A4a} was identified as IgG₂ₐ.

### EXAMPLE 9

### MAb inhibition of P450-catalyzed metabolism

To determine inhibitory activity of the MAb, 10 µl of ascites (or diluted ascites containing inhibitory MAbs) was added to 950 µl of 100 mM KPi (potassium phosphate buffer, pH = 7.5) containing 10-50 pmol of each recombinant P450, or 50-100 pmol of total P450 from liver microsomes of human, monkey, dog, rat and mouse. The mixture was vortexed gently and was preincubated at room temperature for 5 min. The reaction was initiated by the addition of substrate(s) and NADPH (1 mM) in a total volume of 1-mL and incubated at 37°C for 15-30 min depending on substrates used. Incubations were terminated by the addition of 8 vol. of dichloromethane (DCM) and corresponding internal standards; see Table 1. The remaining substrate and metabolites formed were extracted and centrifuged for 10 min (500g). Organic phase was evaporated to dryness under a stream of nitrogen. The residue was dissolved in a mixture of methanol or acetonitrile and water and analyzed immediately by reverse-phase HPLC (HP1100) or LC-MS (Perkin Emer API-150). P450-catalyzed reactions were assayed with slight modifications; see Table 1. The modifications where needed were primarily to change the HPLC conditions including column selection and elution of the gradient to resolve better separation of the metabolites for quantitation.

The assays of phenanthrene-9-hydroxylation (CYP1A1), estrone 2-hydroxylation (CYP1A2), diazepam N-demethylation (CYP2B6), and testosterone 6β-hydroxylation (CYP3A4/5/7) were performed virtually as disclosed; Shou et al., 1994 Cancer Lett. 83:305-313; Shou et al., 1997 Carcinogenesis 18:207-214; Yang et al., 1998 Biochem. Pharmacol. 55:889-896; and Hanioka et al., 1990 Protein Eng. 3:571-575, respectively. For all other assays, separation of metabolites formed was accomplished using a Hewlett Packard model HP1100 liquid chromatograph. The metabolite of taxol 6α-hydroxylation (CYP2C8) was isolated on an ODS Hypersil column (5µ, 4.6 mm x 20 cm, Hewlett Packard) and detected at a UV absorption of 230 nm; Rahman et al., 1994 Cancer Res. 54:5543-5556. The mobile phase for metabolite elution was 10% acetonitrile (ACN) in water for 5-min isocratic and then a 25-min gradient to 65% ACN in water. The metabolite of bufuralol 1'-hydroxylation (CYP2D6) was separated by Zorbax C8 column (5µ, 4.6mm x 15 cm), eluted with 20% ACN in water for 5 min and a 15-min gradient from 20% to 70%, and detected with a fluorescence detector at 252 nm of excitation and 302 nm of emission; Crespi et al., 1995 Pharmacogenetics 5:234-243. The 4'-hydroxy product formed in the metabolism of flurbiprofen was measured on the ODS C18 20/20 column (5µ, 4.6mm x 15 cm) and eluted with a 20-min gradient from 30% ACN in water containing 0.1% acetic acid to 60% at a detection of 260 nm of excitation and 320 nm of emission; Tracy et al., 1995 Biochem. Phamacol. 49:1269-1275. 7-Hydroxycoumarin, a metabolite of coumarin 7-hydroxylation (CYP2A6) was analyzed by HPLC on a column (ODS C18 20/20 column, 5µ, 4.6mm x 15 cm) eluted with a gradient from 40% methanol in water to 70% under a detection of 320 nm; Yun et al., 1991 Mol. Pharmacol.40:679-685. Separation of mephenytoin 4'-hydroxylation (CYP2C19) was performed by using ODS-C18 20/20 column which eluted with a 20-min gradient from 10% ACN in water containing 0.1% acetic acid to 40% and the metabolite was detected by UV absorption of 222 nm; Goldstein et al., 1994 Biochemistry 33:1743-1752. 6-Hydroxychlorzoxazone is a product of chlorzoxazone metabolism by CYP2E1 (Peter et al., 1990 Chem. Res. Toxicol. 3:566-573) and separated on ODS-C18 20/20 column using an elution of a 20-min gradient from solvent A (9% ACN:1% tetrahydrofuran:90% water) to solvent B (79%CAN:1% tetrahydrofuran:9% water) and the detection was 287 nm. Flow rate of HPLC for all assays was 1 mL/min and the quantitation of metabolite was normalized the ratio of the metabolite to internal standard as listed in Table 1. Percentage inhibition of the MAb for the P450-catalyzed metabolism was determined by comparing the rate of metabolite formation between the absence and the presence of the MAb.

### EXAMPLE 10

### MAb_{3A4a} inhibition of CYP3A4/5/7-catalyzed metabolism

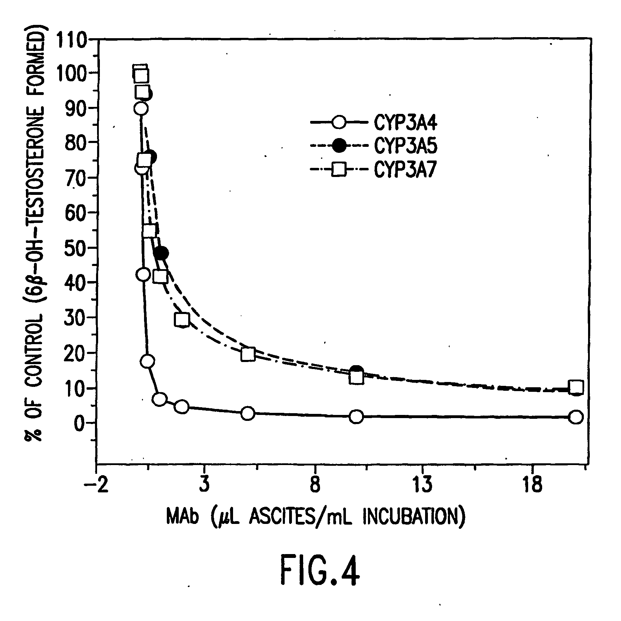

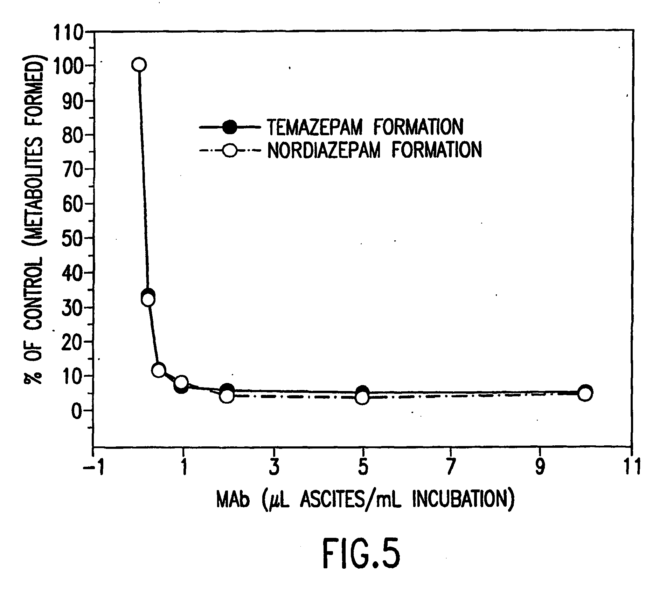

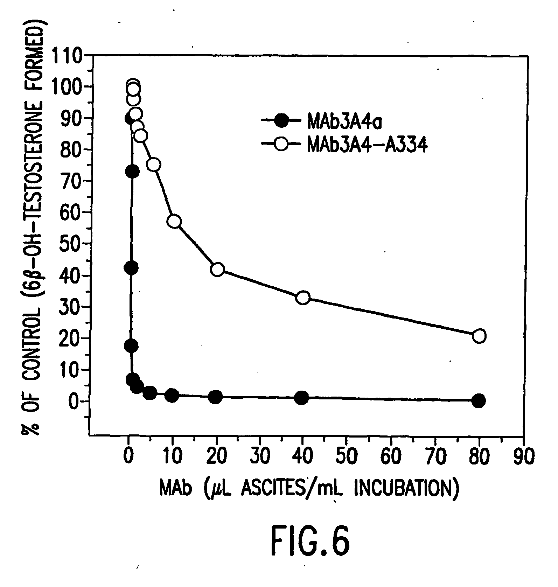

MAb_{3A4a} was examined for its inhibitory effect on CYP3A4-, CYP3A5-, and CYP3A7-catalyzed metabolism of two known substrates, testosterone and diazepam. Figs. 4 and 5 show the titration curve of MAb inhibition of metabolism versus mouse ascites amount. MAb_{3A4a} was found to be very inhibitory to all three CYP3A forms. As seen in Fig. 4, addition of MAb_{3A4a} at 1 µl ascites per one mL incubation of testosterone containing 20 pmol of each enzyme, inhibited CYP3A4/5/7-catalyzed testosterone 6β-hydroxylation by 95%, 52% and 58%, respectively, while at 10 µl/mL, metabolism was inhibited by 99%, 90% and 90%, respectively. Similarly, MAb_{3A4a} (10 µl) inhibited the CYP3A4-mediated conversion of diazepam (DZ) to temazepam (TMZ) and nordiazepam (NDZ) by 99% and 98%, respectively (Fig. 5). In contrast to MAb_{3A4a}, MAb-A334™ (Gentest Corp.) was employed to inhibit CYP3A4-mediated 6β-hydroxylation of testosterone. As seen in Fig. 6, MAb_{3A4a} and MAb-A334™ at 5 µl ascites in one mL incubation inhibited separately CYP3A4 activity by 99% and 24%. When 80 µL ascites of MAb-A334™ was used, only 79% inhibition was observed. These results demonstrate that MAb3A4a is more potent than MAb-A334™.

### EXAMPLE 11

### Cross-inhibition with other human P450s

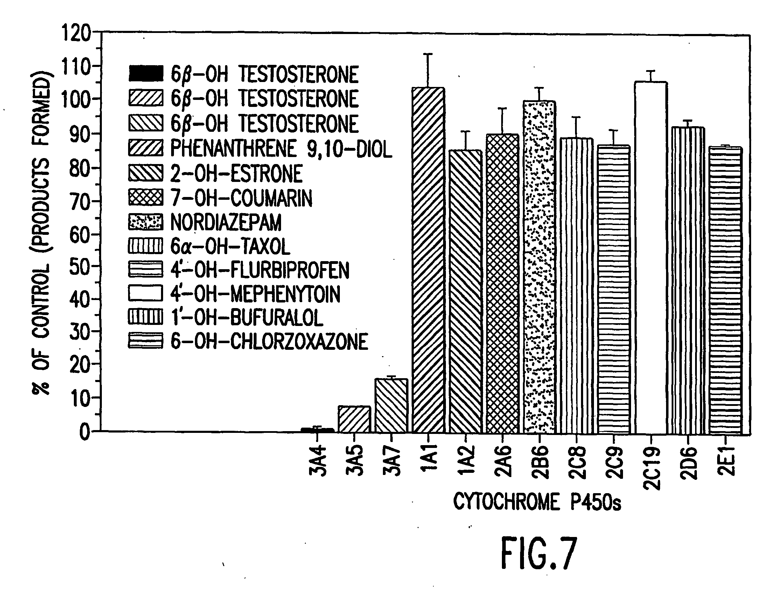

To confirm the specificity of the MAb_{3A4a}, assays for individual cDNA-expressed P450s were performed as shown in Table 1. 10 µl of MAb_{3A4a} ascites was added to one mL of incubation with individual P450s (15-30 pmol per incubation) to examine the cross-inhibition. Fig. 7 shows that MAb_{3A4a} inhibited strongly CYP3A4/5/7-catalyzed testosterone-6β-hydroxylation by 99%, 93% and 94%, respectively but did not display any significantly inhibitory cross-reactivity towards the cDNA-expressed human P450-mediated phenanthrene-9,10-epoxidation (CYP1A1), estrone-2-hydroxylation (CYP1A2), coumarin-7-hydroxylation (CYP2A6), diazepam-*N*-demethylation (CYP2B6), taxol-6α-hydroxylation (CYP2C8), flurbiprofen-4'-hydroxylation (CYP2C9), (S)mephenytoin-4'-hydroxylation (CYP2C19), bufuralol-1'-hydroxylation (CYP2D6) and chlorzoxazone-6-hydroxylation (CYP2E1), respectively, which represented a range of 86% to 108% of the enzyme activity in the absence of the MAb. The results suggest that these P450s may lack the epitope that is recognized by the MAb_{3A4a}.

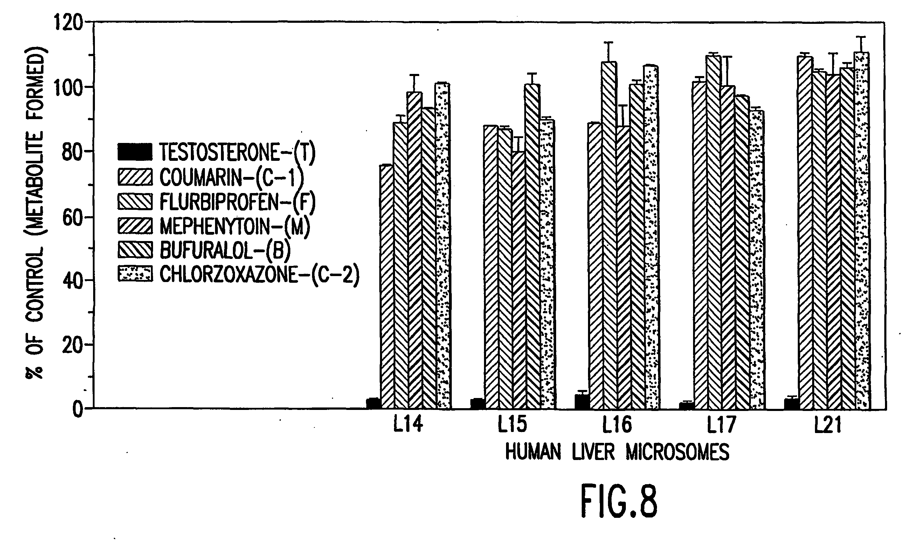

The selectivity of MAb_{3A4a} towards CYP3A4 was also evaluated by human liver microsomes (Fig. 8). Results showed that MAb_{3A4a} inhibited almost exclusively the testosterone-6β-hydroxylation (95∼98%), indicating that the 6β-position as a sole catalytic site for the CYP3A isoforms present in human liver. In contrast to testosterone assay, MAb_{3A4a} did not exhibit any significant effect on coumarin-7-hydroxylation (CYP2A6, 76∼110% of control), flurbiprofen-4'-hydroxylation (CYP2C9, 87∼110%), (S)mephenytoin-4'-hydroxylation (CYP2C19, 80∼104%), bufuralol-1'-hydroxylation (CYP2D6, 92∼115%) and chlorzoxazone-6-hydroxylation (CYP2E1, 89∼111%) (Fig. 8). The minor inhibition exhibited by the MAb_{3A4a} for some marker assays (up to 24%) could be contributed by the overlapping substrate specificity of CYP3A4 in human liver.

To determine whether MAb_{3A4a} inhibition was independent of substrate concentration [S], pooled human liver microsomes were used at varying substrate concentration (relative to Kₘ). Table 2 below shows that MAb_{3A4a} inhibited strongly the CYP3A4 activity (95∼98%) and had no effect on the reactions catalyzed by other P450s. These imply that the MAb-induced inhibition was insensitive to [S] and Kₘ at a saturating concentration of MAb_{3A4a}. As a result, MAb inhibition has an advantage over competitive chemical inhibitors, wherein the inhibitory specificity and potency often depend on the [S] and Kₘ of substrates studied.

**Table 2. MAb_{3A4a} inhibition of the metabolism of marker substrates by human liver microsomes**

| Substrate | Conc.^{a} (µM) | % Control^{b} (+ MAb) | Substrate | Conc. (µM) | % Control (+ MAb) |
|---|---|---|---|---|---|
| Testosterone | 250 | 1.97±0.05 | Bufuralol | 50 | 115.2±0.23 |
| (CYP3A4)^{c} | | | (CYP2D6) | | |
| | 75 | 2.60±0.00 | | 15 | 111.9±9.08 |
| | 25 | 4.55±0.06 | | 5 | 106.8±14.5 |
| Chlorzoxazone | 500 | 104.7±6.37 | Diclofenac | 100 | 108.3±1.16 |
| (CYP2E1) | | | (CYP2C9) | | |
| | 100 | 103.6±0.56 | | 30 | 113.1±1.14 |
| | 20 | 119.8±0.3 | | 10 | 125.7±0.15 |
| Mephenytoin | 400 | 99.5±1.41 | Phenacetin | 1000 | 101.9±1.75 |
| (CYP2C19) | | | (CYP1A2) | | |
| | 120 | 94.6±4.33 | | 150 | 107.5±1.12 |
| | 40 | 96.4±1.99 | | 25 | 113.9±0.91 |

| | | | | | |
|---|---|---|---|---|---|
| ^{a.} Concentration of substrate used for metabolism. ^{b.} % control was determined by a difference of activity between the presence and absence of MAb_{3A4a}. ^{c.} Individual P450 activities were probed with respective marker substrates. | | | | | |

### EXAMPLE 12

### Cross-inhibition in different species.

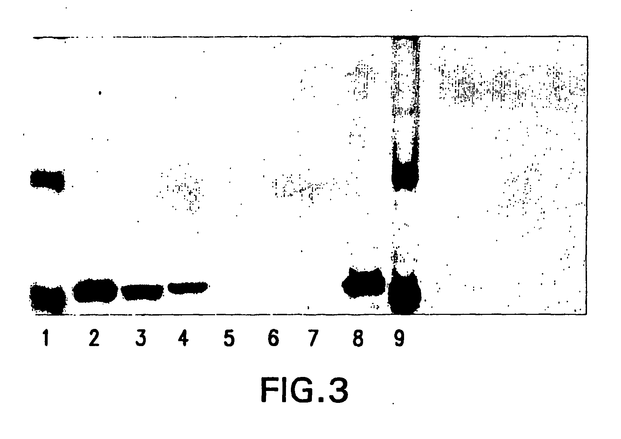

MAb_{3A4a} inhibited the testosterone 6β-hydroxylase activity (94-98%) in liver microsomes from human and Rhesus monkey. By comparison, no inhibition was observed with dog, rat and mouse liver microsomes. This was in agreement with the specificity of MAb_{3A4a} observed with immunoblot assay (Fig. 3).

### EXAMPLE 13

### Quantitative Assessment of CYP3A4/5 Contribution to the Metabolism of Substrates in Human Liver Microsomes.

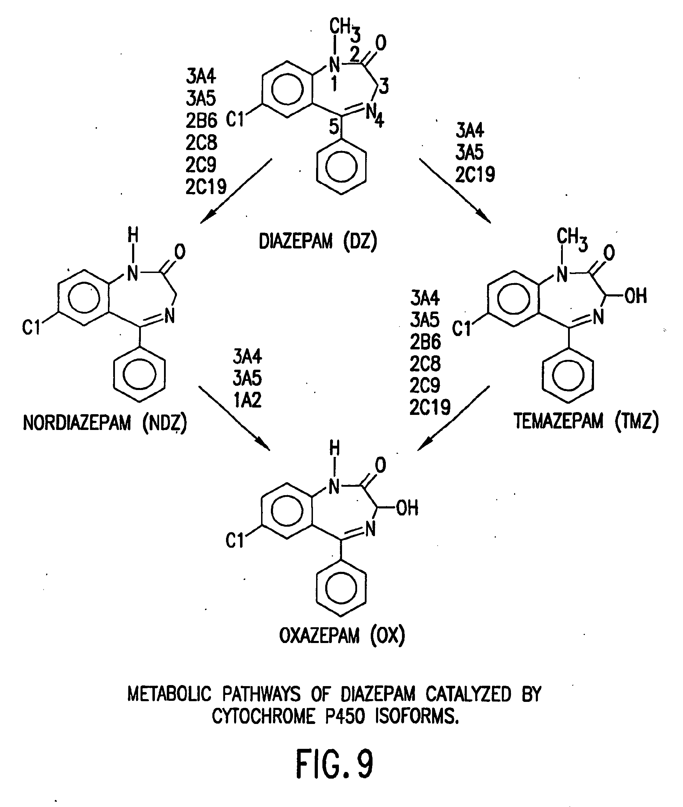

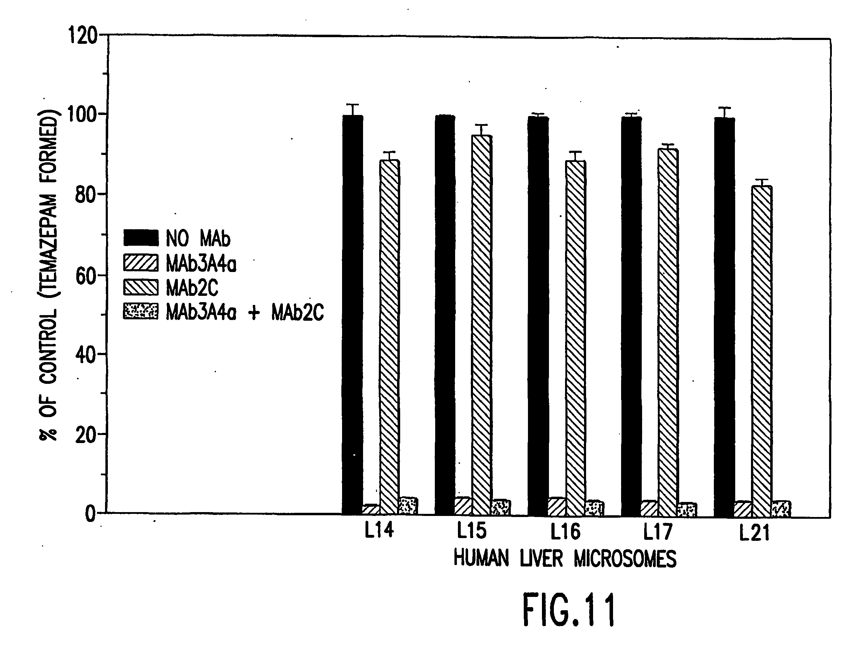

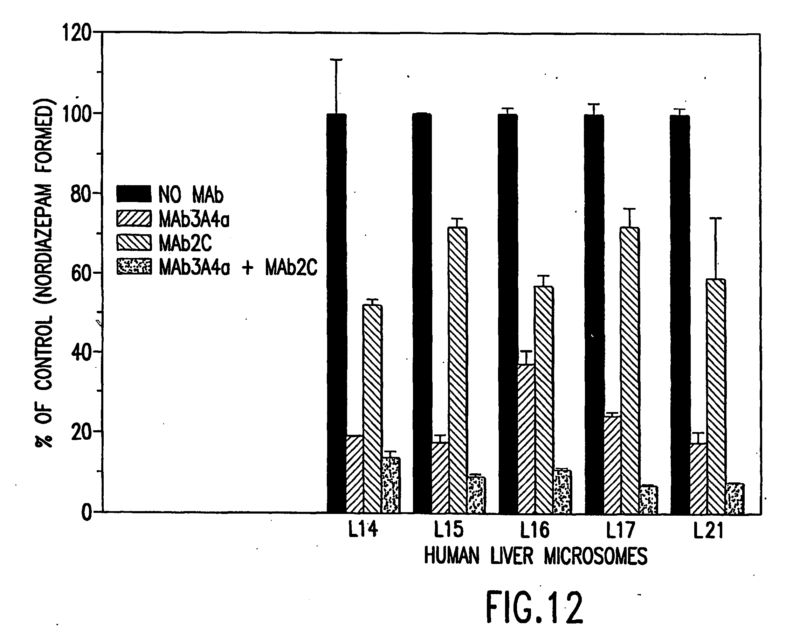

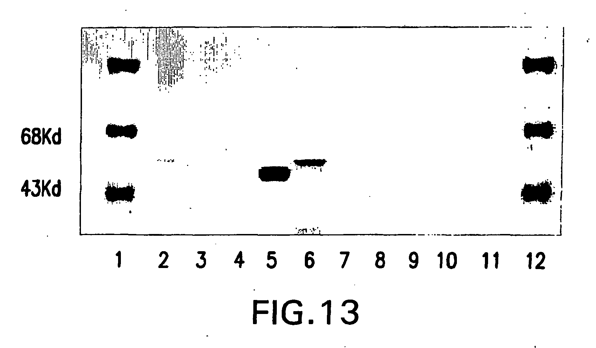

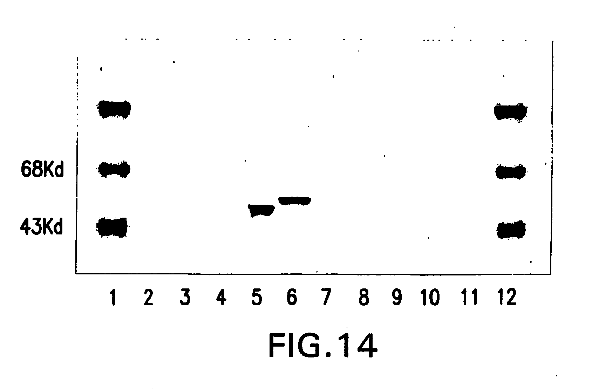

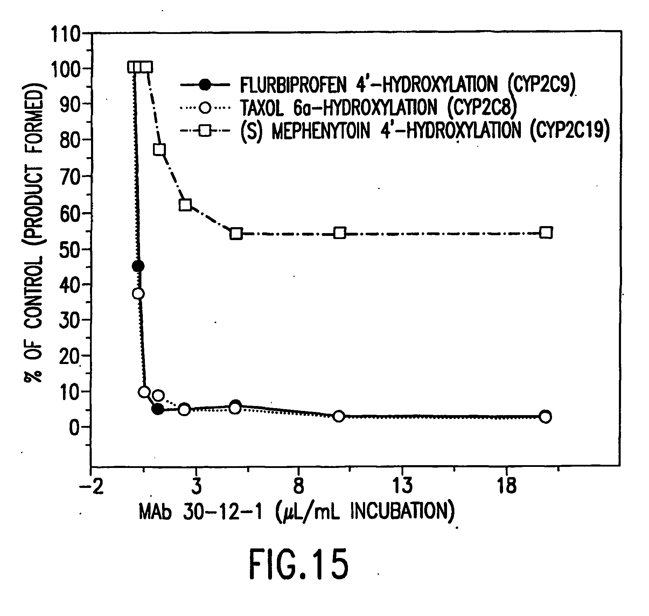

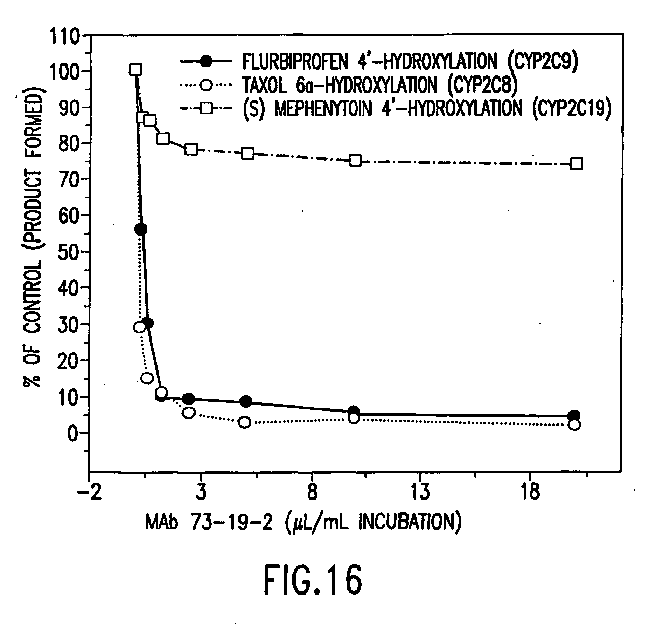

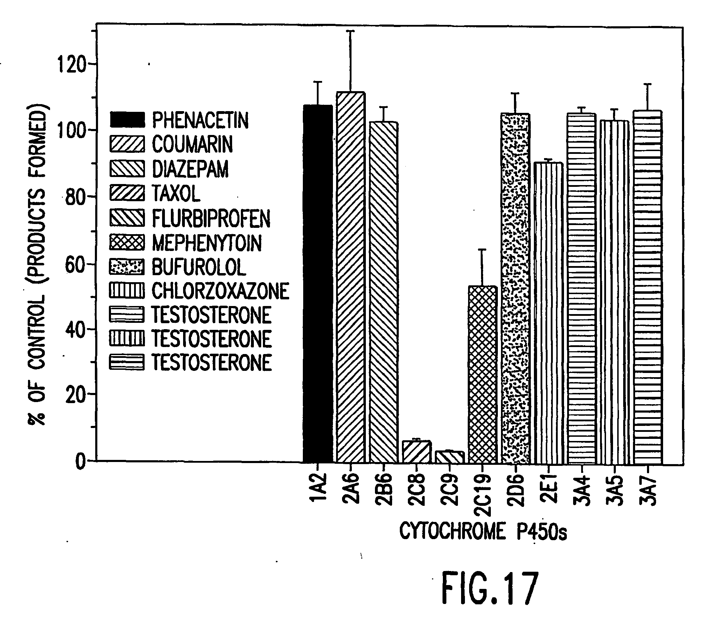

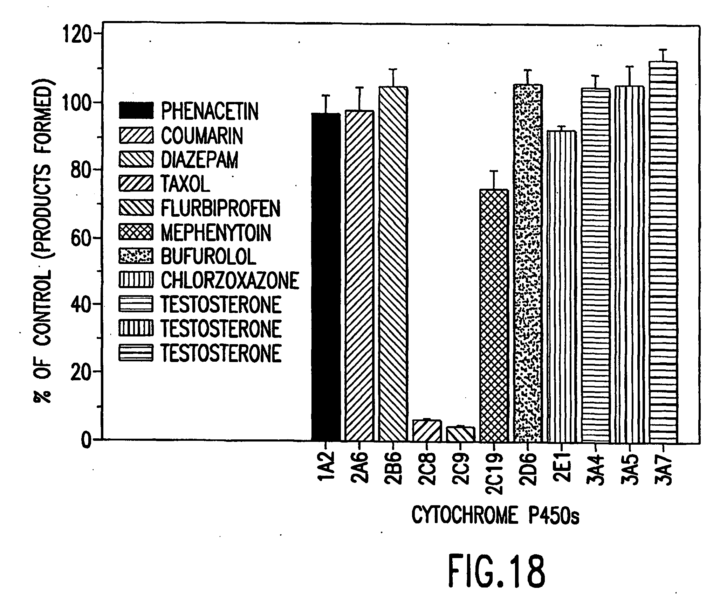

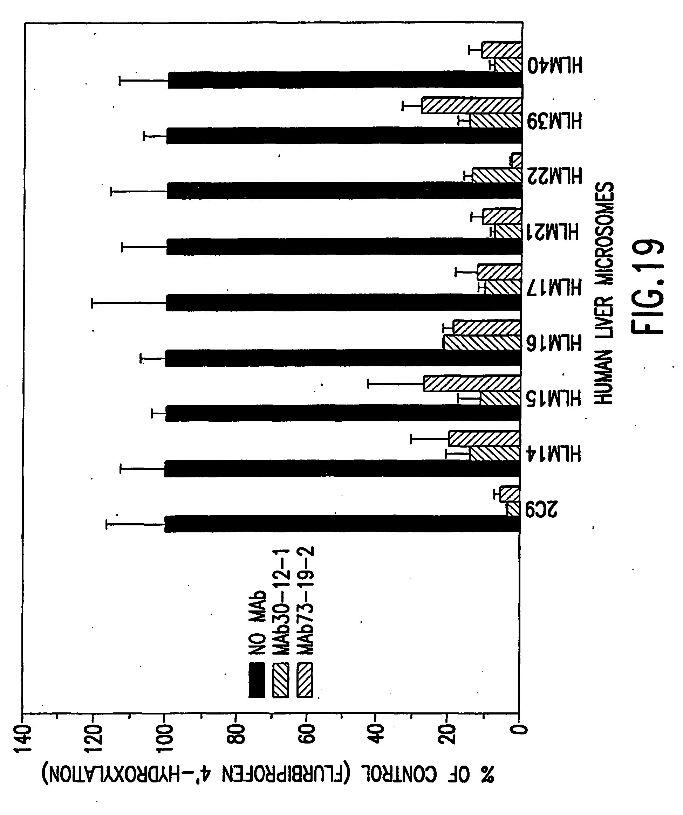

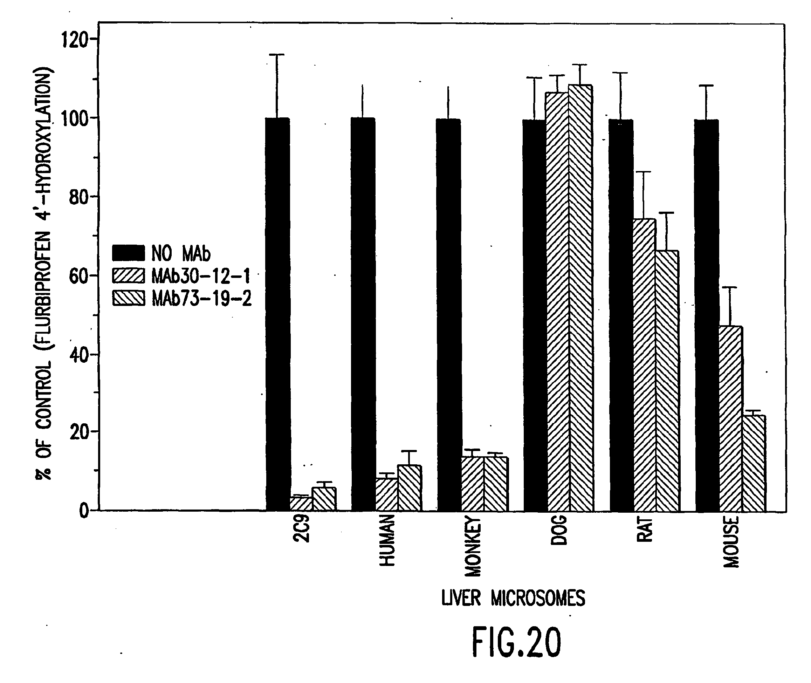

Testosterone is known to be a prototype substrate for CYP3A. MAb_{3A4a} inhibited testosterone 6β-hydroxy formation by more than 95% in all five microsomal preparations, indicating that CYP3A4/5 are essentially the sole P450 responsible for testosterone metabolism in human liver (Fig. 8). Diazepam (DZ) is hydroxylated to temazepam (TMZ) mainly by CYP3A4/5 and *N*-demethylated to nordiazepam (NDZ) by multiple P450s, (e.g., CYP3A4/5, 2B6 and 2C8/9/19) as illustrated in Figure 9. We utilized MAb_{3A4a} and MAb_{2C} (inhibitory anti-CYP2C8/9/19) MAb to assess the additive contribution of the CYP3A and CYP2C subfamilies to TMZ and NDZ formation in human livers. The data indicated that MAb_{3A4a} at 20 □1 of ascites in 1-ml of liver microsomal incubation containing 100 pmol of P450 and 100 □M diazepam, inhibited the conversion of DZ to TMZ by >95% but MAb_{2C} had no apparent effect on TMZ formation (Fig. 11). This indicated that CYP3A4/5 are responsible for the 3-hydrozylation of diazepam in human liver (reaction-specific). However, when the production of nordiazepam was monitored, the reaction was inhibited partially by MAb_{3A4a} (52-73%) and MAb_{2C} (9-37%) (Fig. 12). Interestingly, an additive inhibition was observed in each of 5 liver microsomal samples when both Mabs were used together, showing that 86-93% of the *N*-demethylation of DZ relied on both CYP3A and CYP2C isoforms (Fig. 12). Thus, when a reaction is suspected to be catalyzed by multiple P450s, two or more Mabs can co-incubate in order to evaluate their combined contribution to the metabolism of a particular drug.

## Claims

1. A method of determining expression levels of cytochrome P450 3A4 in a sample which comprises:
(a) contacting the sample with antibody ATCC PTA-351 having a detectable label; and
(b) measuring the amount of label; said amount indicating the level of expression of CYP3A4 in the sample.

2. A method of determining expression levels of cytochrome P450 3A4 in a sample which comprises:
(a) separating a sample on a gel;
(b) transferring the separated sample to a piece of material capable of adsorbing said sample;
(c) contacting the separated sample on the material with a first antibody which is ATCC PTA-351;
(d) contacting the material with a second antibody specific for the first antibody which comprises a detectable label allowing for the quantitation of the bound first antibody; and
(e) quantitating the level of antibody, same indicating the level of expression of cytochrome P450 3A4.

3. A method of determining expression levels of cytochrome P450 3A4 in a sample in accordance with claim 2 wherein:
(a) the gel is an SDS polyacrylamide gel;
(b) the adsorbing material is nitrocellulose paper;
(c) the second antibody is an alkaline phosphatase-conjugated anti-mouse IgG (H+L); and
(d) the quantitating is carried out by treating the alkaline phosphatase-conjugated anti-mouse IgG (H+L) on the adsorbed material with a substrate for the alkaline phosphatase, metabolism of the substrate being identified by a colored product which is to be quantitated.

4. A method of identifying CYP3A4-catalyzed metabolism of a particular substrate, which comprises;
(a) contacting a sample comprising CYP3A4 and antibody ATCC PTA-351 with the substrate;
(b) measuring both substrate and product levels; and
(c) comparing the substrate and product levels with levels obtained from a control sample comprising CYP3A4 and the substrate but not the antibody; a lower level of substrate-to-product conversion in the sample comprising the antibody indicating the presence of CYP3A4-catalyzed metabolism.

5. A method of quantifying CYP3A4-specific metabolism of a substrate in a sample which comprises:
(a) contacting the sample comprising both ATCC PTA-351 antibody and cytochrome P450s known to be involved in the metabolism of the substrate with the substrate;
(b) measuring substrate and product levels;
(c) comparing the substrate and product levels with levels obtained from a control sample comprising both CYP3A4 and the substrate but not the antibody.

6. A monoclonal antibody specific for cytochrome P450 3A4 (CYP3A4) of the isotype IgG₂ₐ which exhibits cross-reactivity with human CYP3A5, human fetal CYP3A7 and monkey testosterone 6β-hydroxylase which is ATCC PTA-351.

## Revendications

1. Méthode de détermination de niveaux d'expression du cytochrome P450 3A4 dans un échantillon qui comprend:
(a) la mise en contact de l'échantillon avec l'anticorps ATCC PTA-351 présentant un marqueur détectable; et
(b) la mesure de la quantité de marqueur; ladite quantité indiquant le niveau d'expression de CYP3A4 dans l'échantillon.

2. Méthode de détermination de niveaux d'expression du cytochrome P450 3A4 dans un échantillon qui comprend:
(a) la séparation d'un échantillon sur un gel;
(b) le transfert de l'échantillon séparé vers un morceau de matériau capable d'adsorber ledit échantillon;
(c) la mise en contact de l'échantillon séparé sur le matériau avec un premier anticorps qui est ATCC PTA-351;
(d) la mise en contact du matériau avec un deuxième anticorps spécifique au premier anticorps qui comprend un marqueur détectable permettant la quantification du premier anticorps lié; et
(e) la quantification du niveau d'anticorps, ce dernier indiquant le niveau d'expression du cytochrome P450 3A4.

3. Méthode de détermination de niveaux d'expression du cytochrome P450 3A4 dans un échantillon selon la revendication 2, dans laquelle:
(a) le gel est un gel SDS polyacrylamide;
(b) le matériau adsorbant est un papier de nitrocellulose;
(c) le deuxième anticorps est une IgG (H+L) anti-souris conjuguée à une phosphatase alcaline; et
(d) la quantification est réalisée par le traitement de la IgG (H+L) anti-souris conjuguée à une phosphatase alcaline sur le matériau adsorbé avec un substrat pour la phosphatase alcaline, le métabolisme du substrat étant identifié par un produit coloré qui est à quantifier.

4. Méthode d'identification d'un métabolisme catalysé par CYP3A4 d'un substrat particulier, qui comprend:
(a) la mise en contact d'un échantillon comprenant CYP3A4 et l'anticorps ATCC PTA-351 avec le substrat;
(b) la mesure des niveaux à la fois de substrat et de produit; et
(c) la comparaison des niveaux de substrat et de produit avec des niveaux obtenus à partir d'un échantillon témoin comprenant CYP3A4 et le substrat mais pas l'anticorps; un niveau inférieur de conversion de substrat en produit dans l'échantillon comprenant l'anticorps indiquant la présence d'un métabolisme catalysé par CYP3A4.

5. Méthode de quantification d'un métabolisme spécifique à CYP3A4 d'un substrat dans un échantillon qui comprend:
(a) la mise en contact de l'échantillon comprenant à la fois l'anticorps ATCC PTA-351 et un cytochrome P450s connu pour être impliqué dans le métabolisme du substrat avec le substrat;
(b) la mesure des niveaux de substrat et de produit;
(c) la comparaison des niveaux de substrat et de produit avec des niveaux obtenus à partir d'un échantillon témoin comprenant à la fois CYP3A4 et le substrat et pas l'anticorps.

6. Anticorps monoclonal spécifique au cytochrome P450 3A4 (CYP3A4) de l'isotype IgG₂ₐ qui affiche une réactivité croisée avec un CYP3A5 humain, un CYP3A7 foetal humain et une 6β-hydroxylase de testostérone de singe qui est ATCC PTA-351.

## Patentansprüche

1. Ein Verfahren zur Bestimmung der Expressionsgrade von Zytochrom P450 3A4 in einer Probe, umfassend:
(a) Inkontaktbringen der Probe mit Antikörper ATCC PTA-351 mit einer nachweisbaren Markierung und
(b) Messen der Menge der Markierung, wobei die Menge den Expressionsgrad von CYP3A4 in der Probe anzeigt.

2. Ein Verfahren zur Bestimmung der Expressionsgrade von Zytochrom P450 3A4 in einer Probe, umfassend:
(a) Auftrennen einer Probe auf einem Gel,
(b) Übertragen der aufgetrennten Probe auf ein Stück Material, das in der Lage ist, die Probe zu adsorbieren,
(c) Inkontaktbringen der aufgetrennten Probe auf dem Material mit einem ersten Antikörper, der ATCC PTA-351 ist,
(d) Inkontaktbringen des Materials mit einem zweiten Antikörper, der für den ersten Antikörper spezifisch ist und der eine nachweisbare Markierung enthält, welche die Quantifizierung des gebundenen ersten Antikörpers ermöglicht, und
(e) Quantifizieren der Menge an Antikörper, welche den Expressionsgrad von Zytochrom P450 3A4 anzeigt.

3. Ein Verfahren zur Bestimmung der Expressionsgrade von Zytochrom P450 3A4 in einer Probe gemäß Anspruch 2, wobei:
(a) das Gel ein SDS-Polyacrylamidgel ist,
(b) das adsorbierende Material Nitrozellulosepapier ist,
(c) der zweite Antikörper ein alkaliphosphatasekonjugiertes Anti-Maus-IgG (H+L) ist, und
(d) die Quantifizierung durch Behandlung des alkaliphosphatasekonjugierten Anti-Maus-IgG (H+L) auf dem adsorbierten Material mit einem Substrat für die Alkaliphosphatase durchgeführt wird, wobei der Metabolismus des Substrats identifiziert wird durch ein gefärbtes Produkt, das zu quantifizieren ist.

4. Ein Verfahren zur Identifizierung des CYP3A4-katalysierten Metabolismus eines speziellen Substrats, umfassend:
(a) Inkontaktbringen einer Probe, die CYP3A4 und Antikörper ATCC PTA-351 enthält, mit dem Substrat,
(b) Messen sowohl des Substrat-Levels als auch des Produkt-Levels und
(c) Vergleich der Substrat- und Produkt-Levels mit Levels, die aus einer Kontrollprobe erhalten wurden, welche CYP3A4 und das Substrat, nicht jedoch den Antikörper umfasst, wobei ein niedrigerer Substrat-Produkt-Umwandlungsgrad in der Probe, welche den Antikörper enthält, die Gegenwart von CYP3A4-katalysiertem Metabolismus anzeigt.

5. Ein Verfahren zur Quantifizierung des CYP3A4-spezifischen Metabolismus eines Substrats in einer Probe, umfassend:
(a) Inkontaktbringen der Probe, die sowohl ATCC-PTA-3 51-Antikörper als auch Zytochrom P450s enthält, von dem bekannt ist, dass es beim Metabolismus des Substrats beteiligt ist, mit dem Substrat,
(b) Messen der Substrat- und Produkt-Levels,
(c) Vergleich der Substrat- und Produkt-Levels mit Levels, die aus einer Kontrollprobe erhalten wurden, welche sowohl CYP3A4 als auch das Substrat, nicht jedoch den Antikörper umfasst.

6. Ein monoklonaler Antikörper spezifisch für Zytochrom P450 3A4 (CYP3A4) des Isotops IgG₂ₐ, der eine Kreuzreaktivität mit menschlichem CYP3A5, menschlichem fötalem CYP3A7 und Affen-Testosteron-6β-Hydroxylase aufweist, welcher ATCC PTA-351 ist.
